# EUROPEAN PATENT APPLICATION

(11) **EP 2 835 093 A1**
(43) Date of publication of application: **11.02.2015**
(21) Application number: 13773011.5
(22) Date of filing: 24.01.2013
(51) Int. Cl.: A61B 1/00, A61B 1/04, A61B 1/06, G02B 23/26

(54) **ENDOSCOPE**

(30) Priority: 05.04.2012 JP 2012086722
(71) Applicant: OLYMPUS CORPORATION, Shibuya-ku Tokyo 151-0072 (JP)
(72) Inventor: NISHIMURA, Yoshiro, Shibuya-ku, Tokyo 1510072 (JP)
(74) Representative: Winter, Brandl, Fürniss, Hübner, Röss, Kaiser, Polte - Partnerschaft mbB
(86) International application number: PCT/JP2013/051409
(87) International publication number: WO 2013/150810

(57) **Abstract**

An endoscope 1 includes illumination units 10 and an image pickup unit 70 that acquires an object image, in a distal end portion 11 of an insertion portion 12. Each the illumination unit 10 includes: a light-emitting section 30 including an LED (31), the light-emitting section 30 outputting light from an upper surface; a wiring board 20 including a recess portion 21 with the light-emitting section 30 disposed therein, and a connection electrode 23 connected to a device electrode 32 of the LED (31) via a relay electrode 22 and an internal wiring 24 in a first principal surface 20SA; a prism 50 disposed above the upper surface of the light-emitting section 30 with a transparent resin layer 40 interposed therebetween, the prism 50 changing a direction of the light outputted by the light-emitting section 30; and a signal cable 60 connected to the connection electrode 23.

## Description

### Technical Field

Embodiments of the present invention relate to an endoscope including an illumination unit in a distal end portion of an insertion portion.

### Background Art

Illumination units using semiconductor light-emitting devices are compact and power-efficient. Therefore, illumination units including semiconductor light-emitting devices such as light-emitting diodes (LED) or laser diodes are used as various types of light sources.

As illustrated in Fig. 1, Japanese Patent Application Laid-Open Publication No. 11-267099 discloses an endoscope 101 in which an illumination unit 110 including LEDs 130 is disposed in a distal end portion 111 of an insertion portion 112. The LEDs 130 disposed on a wiring board 120 emit light according to signals from signal cables 160. The outputted light L reaches an object through an illumination lens 155. The reflected light from the object enters a solid-state image pickup device 171 in an image pickup unit through an objective lens 175, and an image pickup signal is transmitted through signal cables 174. A direction of application of the outputted light L is a direction of image pickup by the image pickup unit, that is, a long axis direction (Z direction) of the distal end portion 111. The signal cables 160 are soldered to electrodes on the principal surface side that faces an LED 130-disposed surface of the wiring board 120.

However, the illumination unit 110 disposed in the distal end portion 111 of the endoscope 101 is extremely small, and thus, it is not easy to perpendicularly connect the signal cables 160 to the principal surface of the wiring board 120. Thus, the endoscope 101 is poor in work efficiency at the time of manufacture of the illumination unit 110.

An object of the present invention is to provide an endoscope that facilitates manufacture of an illumination unit.

### Disclosure of Invention

### Means for Solving the Problem

An endoscope according to an embodiment of the present invention includes an illumination unit and an image pickup unit that acquires an object image, in a distal end portion of an insertion portion. The illumination unit includes: a light-emitting section including a semiconductor light-emitting device, the light-emitting section outputting light from an upper surface; a wiring board including a recess portion with the light-emitting section disposed therein, and a connection electrode connected to a device electrode of the semiconductor light-emitting device via a relay electrode and an internal wiring in either of principal surfaces; an optical member disposed above the upper surface of the light-emitting section with a transparent resin layer interposed therebetween, the optical member changing a direction of light outputted by the light-emitting section; and a signal cable connected to the connection electrode.

### Brief Description of the Drawings

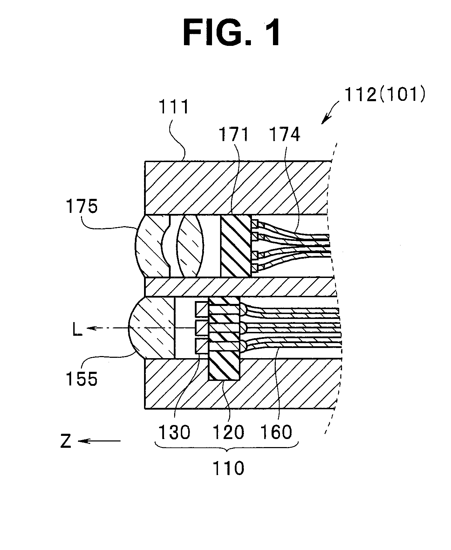

Fig. 1 is a cross-sectional view illustrating a structure of a distal end portion of a conventional endoscope;
Fig. 2 is an outer appearance view for describing a structure of an endoscope according to a first embodiment;
Fig. 3 is an outer appearance view of a distal end face of the endoscope according to the first embodiment;
Fig. 4 is a cross-sectional view illustrating a structure of a distal end portion of the endoscope according to the first embodiment;
Fig. 5 is an exploded view of an illumination unit in the endoscope according to the first embodiment;
Fig. 6 is an outer appearance view of a distal end face of an endoscope according to a modification of the first embodiment;
Fig. 7 is an outer appearance view of a distal end face of an illumination unit in the endoscope according to the modification of the first embodiment;
Fig. 8 is a cross-sectional view illustrating a structure of a distal end portion of an endoscope according to a modification of the first embodiment;
Fig. 9 is a cross-sectional view for describing a structure of an illumination unit in an endoscope according to a second embodiment;
Fig. 10 is a cross-sectional view for describing a structure of an illumination unit in an endoscope according to a modification of the second embodiment;
Fig. 11 illustrates a spectrum of white light produced by the illumination unit in the endoscope according to the modification of the second embodiment; and
Fig. 12 illustrates a spectrum of special light produced by the illumination unit in the endoscope according to the modification of the second embodiment.

### Best Mode for Carrying Out the Invention

### <First Embodiment>

An endoscope 1 according to a first embodiment will be described below with reference to the drawings. Note that each of the drawings is a schematic diagram for description and has, e.g., an aspect ratio that is different from that of the actual dimensions.

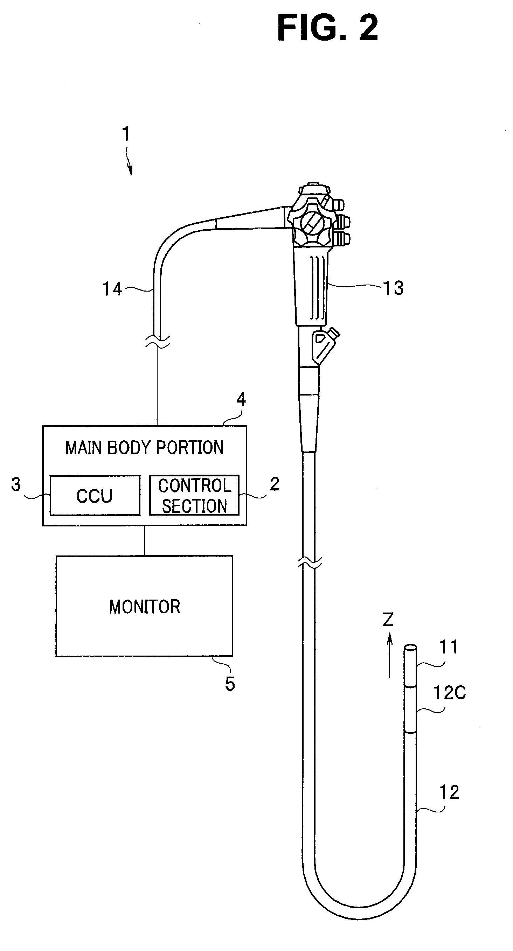

As illustrated in Fig. 2, the endoscope 1 according to the first embodiment includes an insertion portion 12 to be inserted into the body of a subject, an operation portion 13 to be grasped by a surgeon, and a universal cord 14 provided so as to extend from the operation portion 13. The universal cord 14 is connected to a main body portion 4 including, e.g., a control section 2 and a camera control unit (CCU) 3, and a monitor 5 is connected to the main body portion 4. The proximal end portion side of a distal end portion 11 of the insertion portion 12 provides a bending portion 12C that changes a long axis direction (Z direction) of the distal end portion 11 according to an operation of the operation portion 13.

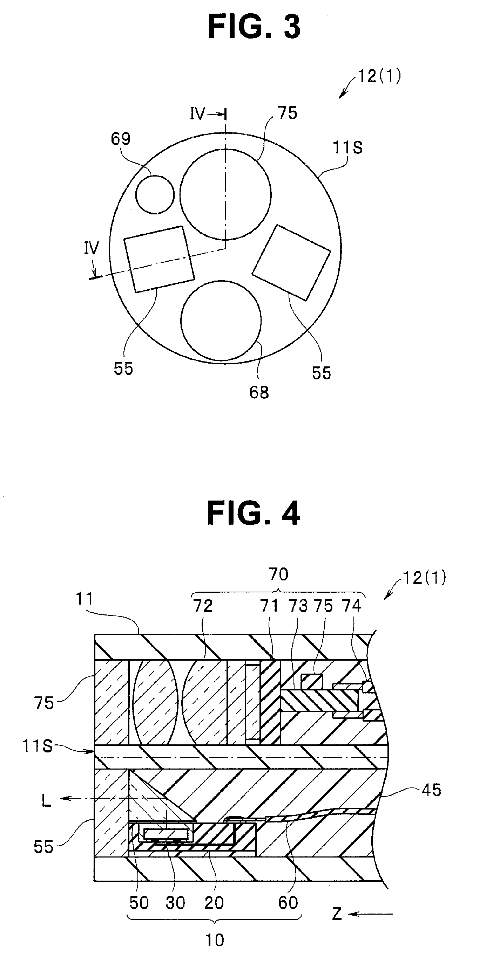

As illustrated in Fig. 3, in a distal end face 11S of the distal end portion 11, rectangular illumination lenses 55, an image pickup lens 76, a forceps port 68 and an air/water feeding port 69 are provided.

Fig. 4 is a cross-sectional view along line IV-IV in Fig. 3. As illustrated in Fig. 4, the endoscope 1 includes an image pickup unit 70 and an illumination unit 10 in the distal end portion 11 of the insertion portion 12. The image pickup unit 70 includes an image pickup section 71, such as a CMOS image sensor, that picks up an object image via a lens group 72 including the image pickup lens 76, which is an outermost lens, a wiring board 73 with a chip component 75 disposed thereon, and a signal cable 74 joined in parallel to a principal surface of the wiring board 73. The illumination unit 10 includes a light-emitting section 30, a wiring board 20, a prism 50, which is an optical member that changes a direction of light outputted by the light-emitting section 30, and signal cables 60 that send a drive signal to the light-emitting section 30. The illumination unit 10 inserted in a hollow portion of the distal end portion 11 is sealed by a sealing resin 45.

Light outputted from an upper surface of the light-emitting section 30, which outputs light from the upper surface, is subjected to direction change by the prism 50 and thereby outputted in a direction (Z direction) that is the same as a direction of image pickup by the image pickup unit 70.

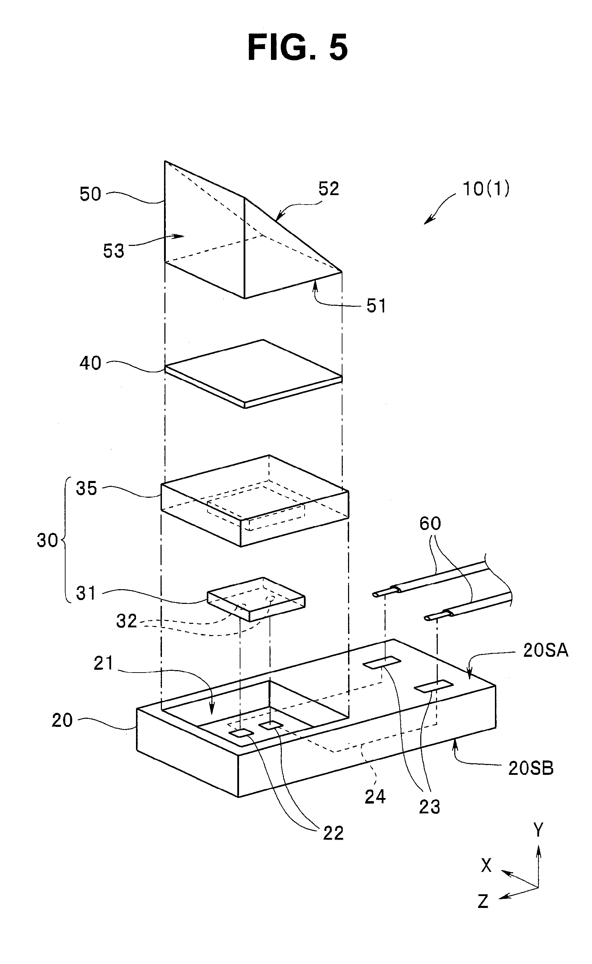

Next, the structure of the illumination unit 10 will be described in detail with reference to Fig. 5. The wiring board 20 includes a first principal surface 20SA and a second principal surface 20SB each having a rectangular shape with a long axis in the Z-axis direction. In the first principal surface 20SA, a recess portion 21 and connection electrodes 23 are formed. A bottom surface of the recess portion 21 is parallel to the principal surfaces 20SA and 20SB of the wiring board 20. In the bottom surface of the recess portion 21, relay electrodes 22 connected to the connection electrodes 23 of the first principal surface 20SA via internal wirings 24 are formed.

Inside the recess portion 21, the light-emitting section 30 including a light-emitting diode (LED) 31, which is a semiconductor light-emitting device, and a wavelength conversion layer 35 is disposed. In other words, the wiring board 20 is a package including high thermal conductivity ceramic, for example, AlN, and housing the LED 31, which is a semiconductor chip.

Note that the illumination unit 10 is extremely small in order to be disposed in the distal end portion 11 of the endoscope 1. For example, a light input surface 51 of the prism 50 has a size of 0.5 mm square, and the LED 21 has a thickness of 0.09 mm and a size of 0.35 mm square, and the wiring board 20 has a size of 2.5 mm in the long axis direction and 1 mm in a short axis direction and a thickness of 0.2 mm.

The device electrodes 32 of the LED 31, which emits blue light (with wavelengths of, for example, 450 to 470 nm), are bonded to the relay electrodes 22 by means of flip-chip bonding. The wavelength conversion layer 35 includes a phosphor-mixed resin that includes a mixture of phosphor particles that upon receipt of blue light, produce yellow light (with wavelengths of, for example, 530 to 550 nm) and a transparent silicone-based resin. Here, the emission wavelengths are indicated by wavelengths in a full width at half maximum.

The prism 50 is disposed above the upper surface of the light-emitting section 30 with a transparent resin layer 40 interposed therebetween. The optical member is not limited to a right angle prism, and may be a reflecting mirror as long as the optical member has a function that changes a direction of light outputted by the light-emitting section 30 by 90 degrees and outputs the light in a long axis direction (Z direction) of the distal end portion 11.

For the transparent resin layer 40, a transparent resin material that does not attenuate light outputted by the light-emitting section 30, for example, a silicone-based resin, an epoxy-based resin or an acrylic-based resin is used.

The signal cables 60 are connected to the connection electrodes 23 in the first principal surface 20SA of the wiring board 20. For the connection, for example, soldering is used, but the connection work to the connection electrodes 23 on a planar surface is easy.

Upon application of a drive signal to the signal cables 60 connected to the connection electrodes 23 of the wiring board 20, white light, which is mixed light of blue light from the LED 31 and yellow light from the wavelength conversion layer 35, is outputted upward (in a Y direction) from the upper surface of the light-emitting section 30. In other words, white light produced by the illumination unit 10 may be pseudo-white light as long as the light is recognized as natural white light to the human eye, and, e.g., a spectrum distribution of the light varies depending on the specifications.

The prism 50, which is an optical member that changes a direction of light outputted by the light-emitting section 30, is disposed on the upper surface of the light-emitting section 30 via the transparent resin layer 40. In other words, the prism 50 totally reflects light inputted from the Y direction perpendicular to the light input surface 51 by means of a reflective surface 52 to output the light forward (in the Z direction) perpendicular to a light output surface 53. As illustrated in e.g., Fig. 4, the Z direction is the long axis direction of the distal end portion 11, and a direction of output of outputted light L is parallel to an optical axis O of the image pickup section 71.

In the endoscope 1 including the illumination units 10 each having the above-described configuration, distal end portions of the cables 60 and the connection electrodes 23 are parallel to each other. In other words, conductive wires of the cables 60 are joined to the first principal surface 20SA, which is a planar part of the wiring board 20, in parallel to the first principal surface 20SA, whereby the manufacture is facilitated.

Next, a method for manufacturing an illumination unit 10 will be described.

First, a wiring board 20 is prepared. The wiring board 20 may be of a metal such as aluminum or copper, glass, a resin such as a glass fiber-contained epoxy resin or polyimide, or any of various types of ceramics. Since the recess portion 21 has an opening having a rectangular shape, the recess portion 21 includes four side walls (side surfaces) and a bottom portion. Depending on the shape of the light-emitting device 31, the opening of the recess portion 21 may have, e.g., a polygonal shape or a circular shape and the wall surfaces may have a tapered shape.

Furthermore, for light extraction efficiency enhancement, the wall surfaces of the recess portion 21 are preferably white or covered by a reflective layer. For providing white wall surfaces, a white base material is used or the wall surfaces are painted white. For the reflective layer (reflector), a highly-reflective film including, e.g., Al, Au or Ni, which is formed by means of vapor deposition or plating, may be used, or the inner walls may be processed to have high reflectivity. Furthermore, if no reflective film is formed on a bottom surface of the LED 31, a reflective film may be formed also on the bottom surface of the recess portion 21 or the bottom surface of the recess portion 21 may be processed to have high reflectivity. Furthermore, a sealing frame or a reflector frame may be provided in an upper portion of the recess portion 21 of the wiring board 20.

Then, the LED 31 is bonded to the bottom surface of the recess portion 21 of the wiring board 20 by means of flip-chip bonding. In other words, device electrodes 32 of the LED 31 and relay electrodes 22 of the wiring board 20 are connected. The LED 31 preferably includes a reflective film on the bottom surface in which the device electrodes 32 are formed, for light extraction efficiency enhancement. The connection between the device electrodes 32 and the relay electrodes 22 may be provided by, e.g., wire bonding or TAB (tape-automated bonding). Also, the LED 31 is not limited to a bare chip-type one, a packaged-type one may be used.

Next, a phosphor-mixed resin is charged in the recess portion 21, that is, the periphery of the LED 31, whereby a wavelength conversion layer 35 is formed. Here, a phosphor-mixed resin may be charged after the periphery of the LED 31 is sealed by, e.g., a transparent resin. Also, e.g., a transparent resin may further be charged on the phosphor-mixed resin charged with a space remaining in the upper portion of the recess portion 21.

For phosphor particles, respective proper phosphors are selected from various types of known fluorescent materials, for example, YAG-based, TAG-based, sialon-based, CaAlSiN₃-based, alkaline earth orthosilicate-based and La oxynitride-based fluorescent materials.

Also, for the light-emitting section 30, any of various types of configurations can be employed as long as such configuration allows the light-emitting section 30 to emit white light. For example, it is possible that an ultraviolet LED is used as an LED and the wavelength conversion layer 35 contains a red phosphor, a green phosphor and a blue phosphor. Also, it is possible to use a blue LED, and a red phosphor and a green phosphor.

Then, a light input surface 51 of a prism 50 is bonded to the wavelength conversion layer 35 on an upper surface of the light-emitting section 30 via a transparent resin layer 40. The transparent resin layer 40 includes, for example, a silicone-based resin.

Note that in order to facilitate the work of bonding the prism 50 including the light input surface 51 of 0.5 mm square, which is extremely small, to the wiring board 20, it is possible to join a support member including a surface parallel to the light input surface 51 to a reflective surface 52 of the prism 50 and dispose the prism 50 at a predetermined position while sucking the support member using a suction nozzle.

Also, it is possible to remove the support member from the prism 50 after the bonding and dispose an electronic component such as a chip capacitor in a space in which the support member had been provided. Also, it is possible to enhance the light extraction efficiency by forming a reflective film on side surfaces of the prism 50. Furthermore, it is possible that: the light-emitting section includes an LED sealed by a transparent resin; and a wavelength conversion layer 35 is disposed on the light input surface 51 or a light output surface 53 of the prism 50.

Then, a plurality of conductive wires of signal cables 60 are connected to a plurality of connection electrodes 23 of the wiring board 20, respectively. As illustrated in Fig. 5, the connection electrodes 23 are formed in a first principal surface 20SA of the wiring board 20 and thus the connection work is easy. Here, the connection electrodes 23 may be formed in a second principal surface 20SB of the wiring board 20. In other words, connection of the signal cables 60 is easy if the connection electrodes 23 are formed in either of the principal surfaces of the wiring board 20.

The illumination unit 10 is fixed to the inside of a hole of a distal end member via an adhesive so that a distal end face 11 S of a distal end portion 11 and the light output surface 53 of the prism 50 are parallel to each other. For heat dissipation efficiency enhancement, it is preferable that a filler having high thermal conductivity be mixed in the adhesive via which the wiring board 20 in the illumination unit 10 is fixed to an inner face of the distal end portion 11 and a sealing resin 45, and for the filler, for example, silica, metal powder, alumina or aluminum nitride is used, and for the resin, e.g., a silicone-based or acrylic-based resin is used.

Furthermore, as a result of disposing the illumination unit 10 in such a manner that an illumination lens 55 is adjacent to an air/water feeding port 69, heat generated in the illumination unit 10 is efficiently dissipated via air/water supplied at the time of air/water feeding. Also, for heat dissipation capability enhancement, e.g., a heat dissipation member formed by bundling metal wires may be attached to the illumination unit 10.

### <Modifications of First Embodiment>

Next, endoscopes 1A and 1B according to modifications 1 and 2 of the first embodiment will be described. Since the endoscope 1A, etc., of the modifications are similar to the endoscope 1 according to the first embodiment, components that are the same as those of the endoscope 1 are provided with reference numerals that are the same as those of the endoscope 1, and description thereof will be omitted.

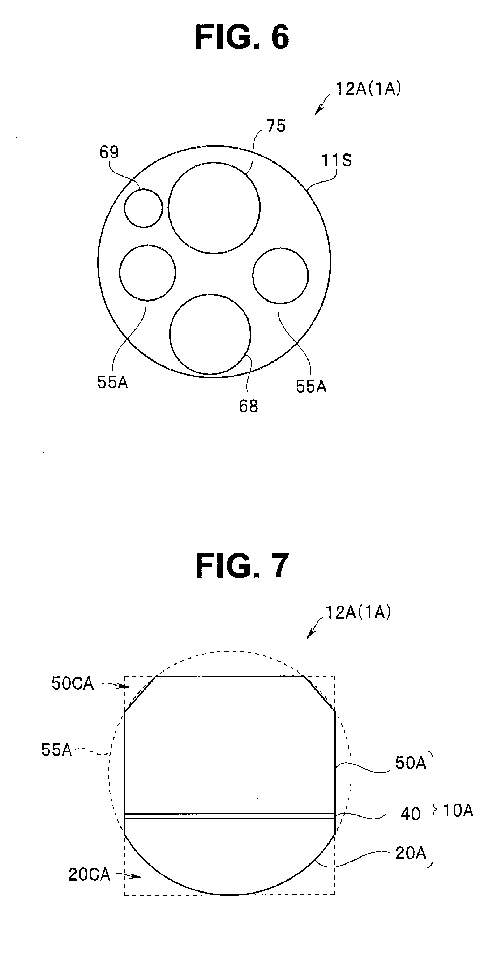

As illustrated in Figs. 6 and 7, in the endoscope 1A according to modification 1, a distal end portion 11 has a cylindrical shape, and corner portions along a long axis direction of each illumination unit 10A are chamfered. In other words, as illustrated in Fig. 6, illumination lenses 55A and 55B in the respective illumination units 10A, which are exposed in a distal end face 11S, have a circular shape, and as illustrated in Fig. 6, corner portions 20CA of each wiring board 20A are chamfered, and corner portions 50CA of each prism 50A are also chamfered. Here, for the shapes resulting from the chamfering, for example, the prism 50A may have a curved surface and the wiring board 20A may have a polygonal shape.

Where holes in the distal end face 11S have a circular shape in cross section, that is, illumination unit disposition holes have a columnar shape, the processing for forming holes can easily be performed compared to cases where the holes have a rectangular shape in cross-section, enabling a distal end member to be manufactured at low cost.

The chamfered illumination units 10A enable easy downsizing of the cylindrical distal end portion 11, and can be disposed inside the respective columnar holes with no gap and thus generated heat is easily transferred to the distal end member.

In other words, the endoscope 1A according to the modification has effects of the endoscope 1, facilitates downsizing and processing of the distal end portion 11 and also provides excellent heat dissipation capability of the illumination units 10A.

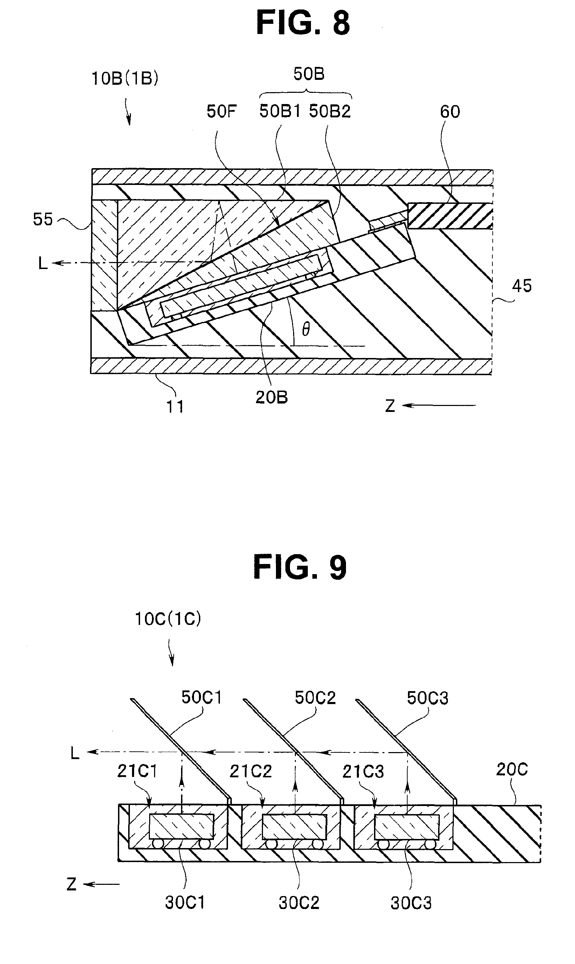

Next, as illustrated in Fig. 8, in each illumination unit 10B in the endoscope 1B according to modification 2, a long axis direction of a wiring board 20B is inclined by an angle θ relative to a long axis direction (Z-axis direction) of a distal end portion. A prism 50B includes two prisms 50B1 and 50B2. The prism 50B1 and the prism 50B2 are bonded via an adhesion layer including a transparent resin having a low refractive index. For example, refractive indices of the prisms 50B1 and 50B1 including high refractive index glass are 1.8, a refractive index of the silicone-based transparent resin is 1.5. Note that in Fig. 8, the adhesion layer is not illustrated.

Outputted light L from an upper surface of a light-emitting section 30B passes through the prism 50B2 and falls on a bonding surface 50F between the prism 50B2 and the prism 50B1; however, since an incident angle of the light is equal to or smaller than a critical angle, the light penetrates the bonding surface 50F and enters the inside of the prism 50B1. The incident light is reflected by an upper surface of the prism 50B1 and then totally reflected because the incident light enters the bonding surface 50F at an angle that is equal to or larger than the critical angle, and is outputted in the long axis direction (Z direction) of the distal end portion 11 of the endoscope 1B.

As the inclination angle θ is larger, a length of the illumination unit 10B can be reduced. The inclination angle θ is preferably 20 to 60 degrees, particularly preferably, 45 degrees from the perspective of design and process.

It is also possible that: a wall surface shape of a hole for disposing the illumination unit 10B in the distal end portion 11 is made to be an inclined shape so as to conform to the shape of the wiring board 20B; and a member having a triangular shape in cross section may be provided between the wiring board 20B and the distal end member. Also, the substrate may be made to have a triangular shape in cross-section.

The endoscope 1B according to the modification has the effects of the endoscope 1 and further facilitates downsizing of the distal end portion 11.

### <Second Embodiment>

Next, an endoscope 1C according to a second embodiment will be described. Since the endoscope 1C is similar to the endoscope 1 according to the first embodiment, components that are the same as those of the endoscope 1 are provided with reference numerals that are the same as those of the endoscope 1 and description thereof will be omitted.

As illustrated in Fig. 9, in each illumination unit 10C in the endoscope 1C, respective light-emitting sections 30C1 to 30C3 are disposed in three recess portions 21C1 to 21 C3 provided in a line in a long axis direction (Z direction) of a wiring board 20C. The light-emitting section 30C1 emits red light, the light-emitting section 30C2 emits green light, and the light-emitting section 30C3 emits blue light. In other words, the three light-emitting sections 30C1 to 30C3 output light of different wavelengths.

Each of the light-emitting sections 30C1 to 30C3 includes an LED 31 that emits an ultraviolet ray (wavelengths: 380 to 420 nm). A wavelength conversion layer 35C1 in the light-emitting section 30C1 contains a red phosphor that upon receipt of ultraviolet light, produces fluorescence of red light (610 to 670 nm). A wavelength conversion layer 35C2 in the light-emitting section 30C2 contains a green phosphor that upon receipt of ultraviolet light, produces fluorescence of green light (500 to 530 nm). A wavelength conversion layer 35C3 in the light-emitting section 30C3 contains a blue phosphor that upon receipt of ultraviolet light, produces fluorescence of blue light (450 to 470 nm).

Then, respective dichroic mirrors 50C1 to 50C3, which each selectively reflect light of the wavelengths emitted by the relevant light-emitting section, are disposed at an angle of 45 degrees on respective upper surfaces of the light-emitting sections 30C1 to 30C3. Accordingly, the illumination unit 10C produces red light, green light and blue light, which are three primary colors of light and thus enables provision of higher color rendering properties than those of the illumination unit 10.

It should be understood that the light-emitting sections 30C1 to 30C3 may include a red LED, a green LED and a blue LED, respectively, and respective transparent resins that seal the respective LEDs.

In the illumination unit 10C, outputted light L from the light-emitting sections 30C1 to 30C3 is outputted along a same optical axis, and particularly, good color rendering properties are provided; however, the plurality of light-emitting sections 30C1 to 30C3 may be provided in a line in a short axis direction of the wiring board 20C. Also, each of the three light-emitting sections 30C1 to 30C3 may be disposed, for example, at a position of an apex of an equilateral triangle on the wiring board.

### <Modification of Second Embodiment>

Next, an endoscope 1D according to a modification of the second embodiment will be described. Since the endoscope 1D according to the modification is similar to the endoscope 1C according to the second embodiment, components that are the same as those of the endoscope 1C are provided with reference numerals that are the same as those of the endoscope 1C, and description thereof will be omitted.

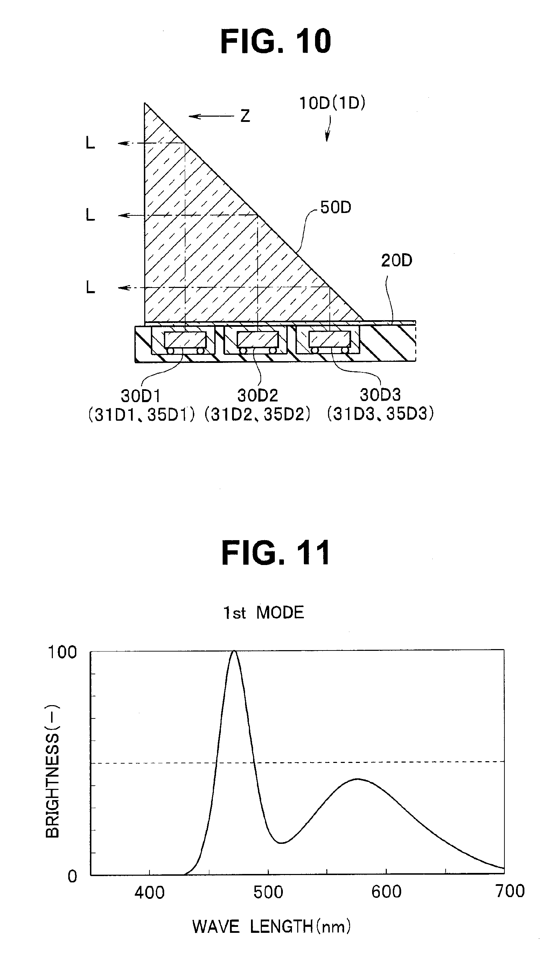

As illustrated in Fig. 10, in each illumination unit 10D in the endoscope 1D, three light-emitting sections 30D1 to 30D3 share one prism 50D. Thus, the endoscope 1D has the effects of the endoscope 1C, and has a large height but a simple structure, and thus can easily be assembled.

Here, regarding endoscopic observation, although normal light observation (white light observation) using visible light has widely been carried out, special light observation utilizing wavelength characteristics of illuminating light has also come into use. For example, in narrow band light observation (narrow band imaging: NBI), in order to observe blood vessels at high contrast, taking particular note of using light having both features of being strongly absorbed by blood and strongly reflected/scattered by a superficial layer of a mucous membrane, display can be provided in such a manner that a contrast between capillary vessels in a superficial layer of a mucous membrane and thick blood vessels in a deep part is enhanced by application of two types of narrow band light.

In the endoscope 1D, the light-emitting sections 30D1 to 30D3 that output light of different wavelengths in the illumination unit 10D can individually be controlled by the control section 2 to be turned on/off.

Thus, as illuminating light, the endoscope 1D can use not only white light for a first mode, but also, special light, for example, light of narrow band wavelengths, as a second mode.

The light-emitting section 30D1 in the illumination unit 10D includes an LED 31D1 that emits ultraviolet light (wavelengths: 400 to 420 nm). The light-emitting section 30D2 includes an LED 31D2 that emits yellow light (wavelengths: 530 to 550 nm). The light-emitting section 30D3 includes an LED 31D3 that emits blue light (wavelengths of 450 to 470 nm). A wavelength conversion layer 35D1 and a wavelength conversion layer 35D2 each include a transparent resin, and a wavelength conversion layer 35D3 in the light-emitting section 30D3 contains a yellow phosphor that produces fluorescence of yellow light (530 to 550 nm).

In the illumination unit 10D, in a first light emission mode (white light observation mode), the light-emitting section 30D3 is on, and the light-emitting section 30D1 and the light-emitting section 30D2 are off. Thus, as illustrated in Fig. 11, white light, which is mixed light of blue light and yellow light, is outputted from the illumination unit 10D.

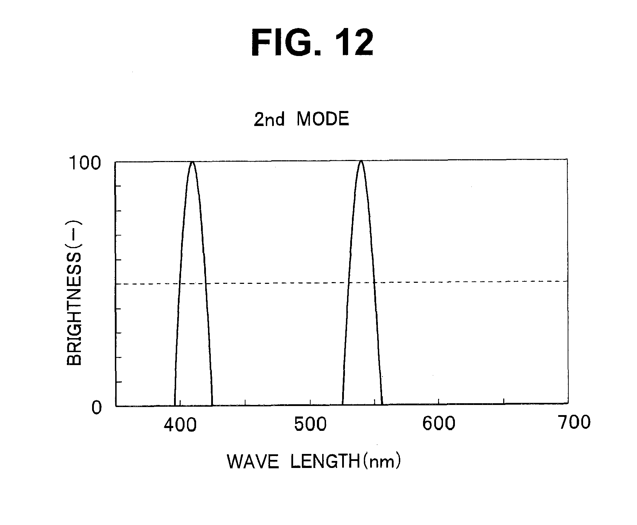

On the other hand, in the illumination unit 10D, in a second light emission mode (white light observation mode), the light-emitting section 30D1 and the light-emitting section 30D2 are on, and the light-emitting section 30D3 is off. Thus, as illustrated in Fig. 12, narrow band light is outputted from the illumination unit 10D.

Note that an endoscope that enables NBI observation may be provided by disposing a filter that transmits only light of predetermined narrow band wavelengths on an optical path of outputted light from a light-emitting section that produces white light in such a manner that transfer the filter in and out can be controlled.

The endoscope 1D has the effects of the endoscope 1C and further enables observations using light in different modes.

Note that two or four or more light-emitting sections may be disposed in each illumination unit in the endoscopes 1C and 1D. Also, as in the modification of the first embodiment, corner portions along the long axis direction may be chamfered or the long axis direction of the wiring board may be inclined by an angle θ relative to a long axis direction (Z-axis direction) of a distal end portion.

Also, like the endoscope 1D according to the modification, the endoscope 1C can be arranged so that the light-emitting sections that output light of different wavelengths can be controlled to be turned on/off individually.

In other words, the present invention is not limited to the above-described embodiments and the like, and various changes, alterations, combinations and the like are possible without departing from the spirit of the present invention.

The present application claims the priority of Japanese Patent Application No. 2012-086722 filed in Japan on April 5, 2012, the disclosure of which is incorporated in the description, the claims and the drawings of the present application by reference.

## Claims

1. An endoscope comprising an illumination unit and an image pickup unit that acquires an object image, in a distal end portion of an insertion portion,
wherein the illumination unit includes:
a light-emitting section including a semiconductor light-emitting device, the light-emitting section outputting light from an upper surface;
a wiring board including a recess portion with the light-emitting section disposed therein, and a connection electrode connected to a device electrode of the semiconductor light-emitting device via a relay electrode and an internal wiring in either of principal surfaces;
an optical member disposed above the upper surface of the light-emitting section with a transparent resin layer interposed therebetween, the optical member changing a direction of light outputted by the light-emitting section; and
a signal cable connected to the connection electrode.

2. The endoscope according to claim 1, wherein a direction of light output by the optical member is a direction of image pickup by the image pickup unit.

3. The endoscope according to claim 2, wherein the light-emitting section is disposed in each of a plurality of recess portions provided in a line along a long axis direction of the wiring board.

4. The endoscope according to claim 3, wherein the respective light-emitting sections disposed in the plurality of recess portions output light of different wavelengths.

5. The endoscope according to claim 4, wherein the light-emitting sections that output light of different wavelengths are individually controlled to be turned on/off.

6. The endoscope according to claim 5, wherein the distal end portion has a cylindrical shape, and a corner portion along a long axis direction of the illumination unit is chamfered.

7. The endoscope according to claim 6, wherein the long axis direction of the wiring board is inclined relative to a long axis direction of the distal end portion.

8. The endoscope according to claim 7, wherein the light outputted by the plurality of light-emitting sections is white light in a first mode, and narrow band light in a second mode, the narrow band light having a band that is narrower than a band of the white light.
